# EUROPÄISCHE PATENTSCHRIFT

(11) **EP 4 347 553 B1**
(45) Veröffentlichungstag und Bekanntmachung des Hinweises auf die Patenterteilung: **23.07.2025**
(21) Anmeldenummer: 22731586.8
(22) Anmeldetag: 01.06.2022
(51) Int. Cl.: C07C 209/68, C07C 211/46, C12P 13/04, C12P 13/00

(54) **VERFAHREN ZUR HERSTELLUNG VON ANILIN ODER EINES ANILINFOLGEPRODUKTES**
METHOD FOR THE PREPARATION OF ANILINE OR AN ANILINE DERIVATIVE
PROCÉDÉ DE FABRICATION D'ANILINE OU D'UN PRODUIT DÉRIVÉ DE L'ANILINE

(30) Priorität: 02.06.2021 EP 21177307
(43) Veröffentlichungstag der Anmeldung: 10.04.2024
(73) Patentinhaber: Covestro Deutschland AG, 51373 Leverkusen (DE)
(72) Erfinder: MACHAT, Martin, 50735 Köln (DE); LANGANKE, Jens, 53894 Mechernich (DE); GÜRTLER, Christoph, 50735 Köln (DE); MARONNA, Marius, 52074 Aachen (DE); GHAZAL, Tofighi, 52074 Aachen (DE); HUSSONG, Christine, 52074 Aachen (DE)
(74) Vertreter: Levpat
(86) Internationale Anmeldenummer: PCT/EP2022/064887
(87) Internationale Veröffentlichungsnummer: WO 2022/253890

(56) Entgegenhaltungen:
- WO-A1-2015/124686
- WO-A1-2018/002088

## Beschreibung

Die dieser Anmeldung zugrunde liegende Erfindung wurde vom deutschen Bundesministerium für Ernährung und Landwirtschaft im Rahmen des Vorhabens "Biobasierte Herstellung von Intermediaten für Polyurethane - Phase II (Bio4PURPro)" (Förderkennzeichen 22019918) finanziell gefördert.

Die vorliegende Erfindung betrifft ein Verfahren zur Herstellung von Anilin oder eines Anilinfolgeproduktes, umfassend die Schritte (I) Bereitstellen von Aminobenzoesäure, (II) Decarboxylieren der Aminobenzoesäure zu Anilin in Gegenwart eines anorganischen heterogenen Metalloxid-Katalysators enthaltend einen auf die Gesamtmasse der Metalloxide bezogenen Massenanteil an Al₂O₃ von 40,0 % bis 100 %, bevorzugt 50,0 % bis 100 %, besonders bevorzugt 60,0 % bis 100 %, wobei der auf die Gesamtmasse des anorganischen heterogenen Metalloxid-Katalysators bezogene Massenanteil an Al₂O₃ 25 % bis 100 % beträgt, und (III) optional, Umsetzen des Anilins zu einem Anilinfolgeprodukt.

Die Herstellung von Anilin durch Decarboxylierung von Aminobenzoesäure ist grundsätzlich im Stand der Technik bekannt. Es sei exemplarisch auf die internationalen Patentanmeldungen WO 2015/124686 A1 und WO 2015/124687 A1 sowie die darin zitierte Literatur verwiesen. Die Ausgangsverbindung Aminobenzoesäure kann chemisch oder vorzugsweise fermentativ gewonnen werden.

Die *chemische* Herstellung von Aminobenzoesäure ist bekannt. Eine geeignete Syntheseroute ist beispielsweise die Reaktion von Phthalimid mit Natriumhypochlorit. Phthalimid kann seinerseits aus Phthalsäureanhydrid und Ammoniak gewonnen werden.

Die *fermentative* Herstellung von Aminobenzoesäure ist ebenfalls bekannt und in der Literatur beschrieben; siehe beispielsweise die bereits erwähnten Anmeldungen WO 2015/124686 A1 und WO 2015/124687 A1 sowie die darin jeweils zitierte Literatur.

WO 2015/124686 A1 beschreibt die Decarboxylierung fermentativ oder chemisch hergestellter Anthranilsäure unter Extraktion von in der Decarboxylierung gebildetem Anilin mit einem systemfremden organischen Lösungsmittel (ein Alkohol, Phenol, Amid, Ether oder aromatischer Kohlenwasserstoff; insbesondere wird 1-Dodecanol als geeignetes Lösungsmittel hervorgehoben). Als Katalysatoren für die Decarboxylierung werden saure Katalysatoren wie Zeolithe oder basische Katalysatoren wie Mg-Al-Hydrotalcit (Mg₆Al₂(CO₃)(OH)₁₆ · 4H₂O, entsprechend einem rechnerischen Massenanteil an "Al₂O₃" von 16,88 %) beschrieben.

WO 2015/124687 A1 beschreibt die Durchführung der Decarboxylierung fermentativ hergestellter Anthranilsäure u. a. in Wasser oder in einem systemfremden organischen Lösungsmittel, insbesondere 1-Dodecanol, gegebenenfalls im Gemisch mit Anilin (vgl. Seite 18, Zeilen 28 und 29). Darüber hinaus beschreibt diese Schrift auch die Möglichkeit der Durchführung der Decarboxylierung in Anilin (ohne 1-Dodecanol; siehe die Abbildungen 35 und 37 bis 38 und die zugehörigen Textstellen), gegebenenfalls in Gegenwart von 10 Massen-% Wasser (siehe die Abbildung 36 und die zugehörigen Textstellen).

In WO 2018/002088 A1 wird ein Verfahren beschrieben, bei welchem Aminobenzoesäure im Gemisch mit Rohanilin decarboxyliert wird. Das Rohanilin stammt aus dem Verfahren selbst, indem ein Teil des Produktstroms nicht der Aufreinigung zugeführt, sondern in das Verfahren rezykliert wird. Als *Katalysatoren* für die Durchführung der Decarboxylierung werden wässrige Säuren wie Schwefelsäure, Salpetersäure und Salzsäure; feste Säuren wie Zeolithe und Si-Ti-Molekularsiebe, feste Basen wie Hydroxy-Apatite und Hydrotalcite; und polymere Säuren wie Ionenaustauscherharze (insbesondere Amberlyst) beschrieben.

In WO 2020/020919 A1 wird die Decarboxylierung von Aminobenzoesäure unter ausschließlicher Verwendung des Produkts Anilin als Katalysator beschrieben. Auf systemfremde Katalysatoren wird bewusst verzichtet.

Keines der zuvor beschriebenen Verfahren ist im Hinblick auf Wirtschaftlichkeit, insbesondere was die Ausbeute an Anilin (Zurückdrängung des Nebenproduktes 2-Aminobenzanilid, wodurch nicht nur der Anteil *gebildeten* Anilins erhöht, sondern auch dessen möglichst quantitative *Isolierung* mittels einer destillativen Reinigung - als Folge verringerter Anilinverluste über die notwendige Hochsiederauschleusung aus dem Sumpf - erleichtert wird) betrifft, völlig zufriedenstellend. Weitere Verbesserungen in der Herstellung von Anilin bzw. Anilinfolgeprodukten durch Decarboxylierung von, insbesondere fermentativ gewonnener, Aminobenzoesäure wären daher wünschenswert.

Dem vorstehend Gesagten Rechnung tragend stellt die Erfindung Folgendes bereit:
Ein Verfahren zur Herstellung von Anilin oder eines Anilinfolgeproduktes, umfassend die Schritte:
(I) Bereitstellen von Aminobenzoesäure (insbesondere ortho-Aminobenzoesäure);
(II) Decarboxylieren der Aminobenzoesäure zu Anilin in Gegenwart eines anorganischen heterogenen Metalloxid-Katalysators (im Folgenden auch kurz als *Katalysator* bezeichnet) enthaltend einen auf die Gesamtmasse der Metalloxide bezogenen Massenanteil an Al₂O₃ von 40,0 % bis 100 %, bevorzugt 50,0 % bis 100 %, besonders bevorzugt 60,0 % bis 100 %, wobei der auf die Gesamtmasse des anorganischen heterogenen Metalloxid-Katalysators bezogene Massenanteil an Al₂O₃ 25 % bis 100 % beträgt;
   und
(III) optional, Umsetzen des Anilins zu einem Anilinfolgeprodukt.

In der Terminologie der vorliegenden Erfindung meint ein *Metalloxidkatalysator* einen Katalysator, der mindestens ein Metalloxid enthält oder der sich formelmäßig als mindestens ein Metalloxid enthaltend darstellen lässt. Die *Gesamtmasse der Metalloxide* bezieht sich auf die maximale Anzahl an Metalloxiden, die formelmäßig darstellbar ist. Lässt sich zum Beispiel die Zusammensetzung eines Katalysators formelmäßig als "Gemisch" aus Aluminiumoxid (Al₂O₃), Magnesiumoxid (MgO) und Wasser (H₂O) darstellen (etwa "*m* Al₂O₃ · *n*MgO · *o* H₂O" *- erste Formel*)*,* so ist es in der Regel auch möglich, denselben Katalysator in einer *zweiten Formel* als "Gemisch" aus Aluminiumhydroxid (Al(OH)₃) oder Aluminium-Oxid-Hydroxid (AlO(OH)) und Magnesiumhydroxid (Mg(OH)₂) zu beschreiben, *wobei weder die eine noch die andere Formel notwendigerweise eine korrekte Wiedergabe der tatsächlichen Struktur ist.* Für die Ermittlung des Massenanteils an Al₂O₃ für *die Zwecke der vorliegenden Erfindung* wird diejenige formelmäßige Beschreibung zugrunde gelegt, welche die maximale Anzahl an Metalloxiden enthält, unabhängig davon, ob die so aufgestellte Formel die tatsächliche Struktur des Katalysators hinreichend wiedergibt oder nicht. Der Massenanteil an Al₂O₃ im Sinne der vorliegenden Erfindung kann daher in diesem Sinne ein rechnerischer Wert sein. Im gewählten Beispiel ist demnach die erste Formel maßgeblich. Für die Massenanteile gegebenenfalls weiterer Metalloxide gilt Entsprechendes.

Es folgt zunächst eine Kurzzusammenfassung verschiedener möglicher **Ausführungsformen der Erfindung,** wobei die Aufzählung an Ausführungsformen nicht als erschöpfend anzusehen ist:
In einer **ersten Ausführungsform** der Erfindung, die mit allen anderen Ausführungsformen kombiniert werden kann, enthält der anorganische heterogene Metalloxid-Katalysator MgO in einem auf die Gesamtmasse der Metalloxide bezogenen Massenanteil von 1,0 % bis 60,0 %, bevorzugt 2,0 % bis 50,0 %, besonders bevorzugt 5,0 % bis 35,0 %.

In einer **zweiten Ausführungsform** der Erfindung, die mit allen anderen Ausführungsformen kombiniert werden kann, enthält der anorganische heterogene Metalloxid-Katalysator SiO₂ in einem auf seine Gesamtmasse bezogenen Massenanteil von 1,0 % bis 30,0 %, bevorzugt 2,0 % bis 20,0 %, besonders bevorzugt 2,0 % bis 10,0 %.

In einer **dritten Ausführungsform** der Erfindung, die mit allen anderen Ausführungsformen kombiniert werden kann, umfasst das Al₂O₃ γ-Al₂O₃ oder η-Al₂O₃, wobei der Katalysator vorzugsweise keine weiteren Metalloxide umfasst.

In einer **vierten Ausführungsform** der Erfindung, die mit allen anderen Ausführungsformen kombiniert werden kann, wird das Decarboxylieren der Aminobenzoesäure bei einer Temperatur von 150 °C bis 300 °C, bevorzugt 160 °C bis 280 °C, ganz besonders bevorzugt 180 °C bis 240 °C durchgeführt.

In einer **fünften Ausführungsform** der Erfindung, die mit allen anderen Ausführungsformen kombiniert werden kann, wird das Decarboxylieren der Aminobenzoesäure bei einem absoluten Druck von 0,05 bar bis 300 bar, bevorzugt 1,0 bar bis 100 bar, besonders bevorzugt 1,0 bar bis 60 bar durchgeführt.

In einer **sechsten Ausführungsform** der Erfindung, die mit allen anderen Ausführungsformen kombiniert werden kann, wird das Decarboxylieren der Aminobenzoesäure in Gegenwart von Anilin durchgeführt.

In einer **siebten Ausführungsform** der Erfindung, die eine besondere Ausgestaltung der sechsten Ausführungsform ist und ansonsten mit allen anderen Ausführungsformen kombiniert werden kann, sofern diese nicht eine kontinuierliche Durchführung der Decarboxylierung vorsehen, wird das Decarboxylieren der Aminobenzoesäure diskontinuierlich durchgeführt, wobei vor Beginn der Decarboxylierung ein Massenanteil an Anilin, bezogen auf die Gesamtmasse an Anilin und Aminobenzoesäure, von 0,1 % bis 90 %, bevorzugt 1,0 % bis 70 %, besonders bevorzugt von 5,0 % bis 50 % eingestellt wird.

In einer **achten Ausführungsform** der Erfindung, die eine weitere besondere Ausgestaltung der sechsten Ausführungsform ist und ansonsten die mit allen anderen Ausführungsformen kombiniert werden kann, sofern diese nicht eine diskontinuierliche Durchführung der Decarboxylierung vorsehen, wird das Decarboxylieren der Aminobenzoesäure kontinuierlich durchgeführt, wobei während der Decarboxylierung stets ein Massenanteil an Anilin, bezogen auf die Gesamtmasse an Anilin und Aminobenzoesäure, von 0,1 % bis 90 %, bevorzugt 1,0 % bis 70 %, besonders bevorzugt 5,0 % bis 50 % eingestellt wird.

In einer **neunten Ausführungsform** der Erfindung, die mit allen anderen Ausführungsformen kombiniert werden kann, wird das Decarboxylieren der Aminobenzoesäure
- in der Flüssig- oder Gasphase in einem Reaktor (insbesondere in einem Rohrreaktor) mit einem integrierten Festbett des anorganischen heterogenen Metalloxid-Katalysators (beinhaltend eine Schüttung des Katalysators als Formkörper (Extrudate) oder eine Ausgestaltung des Katalysators als monolithische Struktur),
- in der Flüssig- oder - bevorzugt - Gasphase in einem Wirbelschichtreaktor oder
- in der Flüssigphase in einem Rührkessel mit einer darin enthaltenen Suspension (*Slurry*) des anorganischen heterogenen Metalloxid-Katalysators
   durchgeführt.

In einer **zehnten Ausführungsform** der Erfindung, die eine besondere Ausgestaltung der neunten Ausführungsform ist, wird das Decarboxylieren der Aminobenzoesäure in der Flüssig- oder Gasphase in einem Reaktor (insbesondere in einem Rohrreaktor) mit einem integrierten Festbett des anorganischen heterogenen Metalloxid-Katalysators beinhaltend eine Schüttung des Katalysators als Formkörper (Extrudate) oder eine Ausgestaltung des Katalysators als monolithische Struktur durchgeführt, wobei der anorganische heterogene Metalloxid-Katalysator nach erfolgter Decarboxylierung regeneriert und wiederverwendet wird.

In einer **elften Ausführungsform** der Erfindung, die mit allen anderen Ausführungsformen kombiniert werden kann, umfasst Schritt (I) das Fermentieren eines Rohstoffes enthaltend eine fermentierbare Kohlenstoff-haltige Verbindung und eine Stickstoff-haltige Verbindung in Gegenwart von Mikroorganismen.

In einer **zwölften Ausführungsform** der Erfindung, die eine besondere Ausgestaltung der elften Ausführungsform ist, umfasst die fermentierbare Kohlenstoff-haltige Verbindung Stärkehydrolysat, Zuckerrohrsaft, Zuckerrübensaft, Hydrolysate aus Lignocellulosehaltigen Rohstoffen oder eine Mischung von zwei der mehr der vorgenannten, wobei die Stickstoff-haltige Verbindung Ammoniakgas, Ammoniakwasser, Ammoniumsalze, Harnstoff oder eine Mischung von zwei oder mehr der vorgenannten umfasst.

In einer **dreizehnten Ausführungsform** der Erfindung, die eine besondere Ausgestaltung der elften und zwölften Ausführungsform ist, umfassen die Mikroorganismen *Escherichia coli, Pseudomonas putida, Corynebacterium glutamicum, Ashbya gossypii, Pichia pastoris, Hansenula polymorpha, Yarrowia lipolytica, Zygosaccharomyces bailii* oder *Saccharomyces cerevisiae.*

In einer **vierzehnten Ausführungsform** der Erfindung, die mit allen anderen Ausführungsformen kombiniert werden kann, wird Schritt (III) durchgeführt und umfasst eine der folgenden Umsetzungen:
(1) Säurekatalysierte Reaktion des Anilins mit Formaldehyd unter Bildung von Di-und Polyaminen der Diphenylmethanreihe;
(2) Säurekatalysierte Reaktion des Anilins mit Formaldehyd unter Bildung von Di-und Polyaminen der Diphenylmethanreihe, gefolgt von deren Umsetzung mit Phosgen unter Bildung von Di- und Polyisocyanaten der Diphenylmethanreihe;
   oder
(3) Umsetzung des Anilins zu einer Azoverbindung.

In einer **fünfzehnten Ausführungsform** der Erfindung, die mit allen anderen Ausführungsformen kombiniert werden kann, umfasst die in Schritt (A) bereitgestellte Aminobenzoesäure ortho-Aminobenzoesäure (Anthranilsäure) und ist insbesondere *ortho-*Aminobenzoesäure (umfasst also keine weiteren Isomere der Aminobenzoesäure).

Die zuvor kurz geschilderten Ausführungsformen und weitere mögliche Ausgestaltungen der Erfindung werden im Folgenden näher erläutert. Dabei sind sämtliche Ausführungsformen und weitere Ausgestaltungen der Erfindung, sofern sich aus dem Kontext für den Fachmann nicht eindeutig das Gegenteil ergibt oder explizit etwas anderes gesagt wird, beliebig miteinander kombinierbar.

Die in **Schritt (I)** zu bereitzustellende Aminobenzoesäure kann grundsätzlich auf jede in der Fachwelt bekannte Art gewonnen werden. Eine Möglichkeit ist die Herstellung der Aminobenzoesäure **auf chemischem Wege.** Bevorzugt sind solche Verfahren, die selektiv das ortho-Isomer der Aminobenzoesäure liefern. Als eine geeignete chemische Methode sei beispielhaft die Reaktion von Phthalimid mit Natriumhypochlorit erwähnt. Phthalimid kann seinerseits aus Phthalsäureanhydrid und Ammoniak gewonnen werden.

Die Herstellung von para-Aminobenzoesäure auf chemischem Wege kann über die Nitrierung von Toluol mit Salpetersäure, anschließende Oxidation des erhaltenen para-Nitrotoluols mit Sauerstoff zu para-Nitrobenzoesäure und schließlich Reduktion zu para-Aminobenzoesäure mit Hydrazin erfolgen.

Die Herstellung von meta-Aminobenzoesäure gelingt beispielsweise ausgehend von Benzoesäuremethylester: Durch Nitrierung von Benzoesäuremethylester mit Salpetersäure wird meta-Nitrobenzoesäuremethylester erhalten. Dieser Methylester wird anschließend mit Natronlauge verseift. Nach Neutralisation mit Salzsäure wird meta-Nitrobenzoesäure erhalten, die schließlich mit Hydrazin zu meta-Aminobenzoesäure reduziert wird.

Erfindungsgemäß ist es jedoch bevorzugt, die für die Durchführung von Schritt (I) erforderliche Aminobenzoesäure durch einen **fermentativen Prozess** herzustellen. In dieser Ausführungsform der Erfindung umfasst das Bereitstellen Aminobenzoesäure die Fermentierung eines Rohstoffes umfassend wenigstens eine fermentierbare Kohlenstoff-haltige Verbindung und eine Stickstoff-haltige Verbindung unter Verwendung von Mikroorganismen unter Erhalt einer Aminobenzoat und/oder Aminobenzoesäure enthaltenden Fermentationsbrühe. Dieser Schritt kann nach einem beliebigen aus dem Stand der Technik bekannten, für die Herstellung von Aminobenzoesäure geeigneten, Fermentationsverfahren durchgeführt werden.

Unter einer *fermentierbaren Kohlenstoff-haltigen Verbindung* im Sinne dieser Ausführungsform der vorliegenden Erfindung wird jede organische Verbindung oder Mischung organischer Verbindungen verstanden, die von den rekombinanten Zellen des eingesetzten Mikroorganismus verwendet werden kann, um Aminobenzoesäure zu produzieren. Die Produktion von Aminobenzoesäure kann dabei in Gegenwart oder Abwesenheit von Sauerstoff stattfinden. Bevorzugt sind dabei solche fermentierbaren Kohlenstoff-haltigen Verbindungen, die zusätzlich als Energie- und Kohlenstoffquelle für das Wachstum der rekombinanten Zellen des eingesetzten Mikroorganismus dienen können. Besonders geeignet sind Stärkehydrolysat, Zuckerrohrsaft, Zuckerrübensaft und Hydrolysate aus lignocellulosehaltigen Rohstoffen sowie Mischungen derselben (d. h. Mischungen aus zwei oder mehr der vorgenannten Verbindungen). Ebenfalls geeignet sind Glycerin und C1-Verbindungen, insbesondere Kohlenstoffmonoxid. Als für Schritt(I)(1) geeignete *Stickstoff-haltige Verbindungen* kommen insbesondere Ammoniakgas, Ammoniakwasser, Ammoniumsalze (insbesondere anorganische Ammoniumsalze wie Ammoniumchlorid und/oder Ammoniumsulfat, bevorzugt Ammoniumsulfat), Harnstoff oder Mischungen derselben (d. h. Mischungen aus zwei oder mehr der vorgenannten Verbindungen) in Frage.

Bevorzugte Mikroorganismen für die Durchführung der Fermentation sind **Bakterien** oder **Pilze,** und zwar insbesondere **Hefen.** Besonders bevorzugt sind dabei Mikroorganismen wie *Escherichia coli, Pseudomonas putida, Corynebacterium glutamicum, Ashbya gossypii, Pichia pastoris, Hansenula polymorpha, Kluyveromyces marxianus, Yarrowia lipolytica, Zygosaccharomyces bailii* oder *Saccharomyces cerevisiae,* wobei der alleinige Einsatz von *Corynebacterium glutamicum, insbesondere Corynebacterium glutamicum ATCC 13032,* besonders bevorzugt ist. Der in der Fermentation einzuhaltende pH-Wert richtet sich nach dem eingesetzten Mikroorganismus. Mikroorganismen wie *Corynebacterium glutamicum, Pseudomonas putida* oder *Escherichia coli* werden bevorzugt bei neutralen pH-Werten (d. h. bei einem pH-Wert im Bereich von 6,0 bis 8,0) kultiviert. Mikroorganismen wie *Saccharomyces cerevisiae* werden hingegen bevorzugt im sauren Milieu kultiviert (d. h. bei einem pH-Wert im Bereich von 3,0 bis 6,0).

In jedem Fall wird der Mikroorgansimus der Fermentation bevorzugt so ausgewählt, dass in der Fermentation das ortho-Isomer von Aminobenzoesäure gebildet wird.

In einer bevorzugten Ausgestaltung der Erfindung werden **Bakterien als Mikroorganismen** eingesetzt. Dabei wird insbesondere auf die Patentanmeldungen WO 2015/124686 A1 und WO 2015/124687 A1 verwiesen, in denen eine erfindungsgemäß einsetzbare Fermentation unter Einsatz von Bakterien beschrieben ist (siehe beispielsweise WO 2015/124687 A1, (i) Seite 15, Zeile 8 bis Seite 16, Zeile 30, (ii) Beispiel 1 (Seite 29, Zeile 4 bis 26), (iii) Beispiel 3 (vor allem Seite 34, Zeile 10 bis 18), (iv) Beispiel 4 (vor allem Seite 55, Zeile 9 bis 31). Insbesondere kommen Bakterien zum Einsatz, die in der Lage sind, eine fermentierbare Kohlenstoff-haltige Verbindung in Gegenwart einer geeigneten Stickstoffquelle in Aminobenzoesäure umzuwandeln, ohne dass die so gebildete Aminobenzoesäure in zellinternen biochemischen Prozessen gleich wieder verbraucht wird, sodass sich Aminobenzoesäure in der Zelle anreichert und schließlich in die Fermentationsbrühe übergeht.

In einer anderen bevorzugten Ausgestaltung der Erfindung werden **Hefen als Mikroorganismen** eingesetzt. Dabei wird insbesondere auf die internationale Anmeldung WO 2017/102853 A1 verwiesen. Insbesondere kommen Hefezellen zum Einsatz, die in der Lage sind, eine fermentierbare Kohlenstoff-haltige Verbindung in Gegenwart einer geeigneten Stickstoffquelle in Aminobenzoesäure umzuwandeln, ohne dass die so gebildete Aminobenzoesäure in zellinternen biochemischen Prozessen gleich wieder verbraucht wird, sodass sich Aminobenzoesäure in der Zelle anreichert und schließlich in die Fermentationsbrühe übergeht.

Geeignete Bakterien oder Hefezellen können beispielsweise durch Screening nach Mutanten, welche Aminobenzoesäure in das umgebende Medium abgeben, identifiziert werden. Die zielgerichtete Modifikation von Schlüsselenzymen mittels gentechnischer Verfahren ist jedoch bevorzugt. Mit üblichen gentechnischen Methoden können Genexpression und Enzymaktivität nach Belieben verstärkt, verringert oder sogar ganz unterbunden werden. Es resultieren rekombinante Stämme.

In der Mehrzahl der Fälle ist die am Ende der Fermentation vorliegende Fermentationsbrühe basisch bis neutral oder allenfalls leicht sauer (pH > 4,7), und die Aminobenzoesäure liegt infolgedessen in Form ihres Anions Aminobenzoat gelöst vor. In diesen Fällen ist es bevorzugt, die Fermentationsbrühe mit Säure zu behandeln, und zwar insbesondere mit Salzsäure, Schwefelsäure und/oder Phosphorsäure, um das Anion in die elektroneutrale Form zu überführen. Die Säurezugabe erfolgt dabei insbesondere solange, bis der pH-Wert der resultierenden Mischung im Bereich von 3,0 bis 4,7, bevorzugt im Bereich von 3,2 bis 3,7 (insbesondere bei meta- und para-Aminobenzoesäure), besonders bevorzugt im Bereich von 3,4 bis 3,6 (insbesondere bei ortho-Aminobenzoesäure), liegt. Dann liegt Aminobenzoesäure überwiegend bis vollständig in der elektroneutralen Form vor und fällt infolge der geringen Wasserlöslichkeit derselben bis auf einen kleinen, auf eine gewisse Restlöslichkeit zurückzuführenden Anteil, aus und kann leicht von der überstehenden Fermentationsbrühe abgetrennt werden, insbesondere durch Filtration oder Zentrifugation. Eine Filtration kann bei vermindertem Druck, Umgebungsdruck oder erhöhtem Druck durchgeführt werden. Eine Zentrifugation kann mit handelsüblichen Zentrifugen durchgeführt werden. Es ist auch möglich, die in der Säurebehandlung erhaltene Suspension ruhen zu lassen, bis sich die ausgefallenen Kristalle an Aminobenzoesäure absetzen, und dann die überstehende Mutterlauge abzudekantieren oder abzusaugen.

Sollte die Fermentationsbrühe jedoch stark sauer (pH < 3,0) sein, wird ein pH-Wert in den vorgenannten Bereichen durch Zugabe von Base (bevorzugt Natronlauge, Kalk) sichergestellt. Liegt der pH-Wert der Fermentationsbrühe dagegen im Bereich von 3,0 bis 4,0, wie es beim Einsatz von Hefen als Mikroorganismen der Fall sein kann, wird in einer bevorzugten Ausführungsform weder Säure noch Base zugegeben, sondern die Fermentationsbrühe wird direkt ohne weitere pH-Wert-Anpassung weiter verarbeitet. In diesem Fall ist damit zu rechnen, dass Kristalle von Aminobenzoesäure spontan ausfallen und direkt abgetrennt werden können. Bezüglich der hierzu anwendbaren Methoden gilt das oben bei der Säurebehandlung Gesagte entsprechend.

Eine erforderlichenfalls nötige Trennung von in wässriger Lösung vorliegender fester Aminobenzoesäure und festen Mikroorganismen gelingt am besten durch Zentrifugation. Dies gilt für alle Ausführungsformen der vorliegenden Erfindung, in denen eine solche Trennung erforderlich ist.

Die auf eine der vorstehend beschriebenen Weisen erhaltene Aminobenzoesäure kann vor der Durchführung der Decarboxylierung weiter aufbereitet werden. Bevorzugt ist eine Wäsche mit wässrigen Waschmedien, insbesondere Wasser. Um Ausbeuteverluste zu vermeiden, kann der pH-Wert des wässrigen Waschmediums auf den gleichen Wert wie nach beendeter Säurezugabe (bzw. im Fall von Hefen: Basenzugabe) eingestellt werden; es wird also in dieser Ausführungsform statt mit Wasser mit einer verdünnten Säure gewaschen. Als Säuren hierfür eignen sich die oben im Zusammenhang mit der Säurebehandlung genannten Säuren.

Die so chemisch oder fermentativ bereitgestellte Aminobenzoesäure wird in **Schritt (II) zu** Anilin decarboxyliert. Der hierzu eingesetzte Katalysator zeichnet sich erfindungsgemäß durch einen hohen Aluminiumoxidmassenanteil aus (mindestens 40 %, ermittelt wie weiter oben beschrieben). Bei dem Aluminiumoxid handelt es sich vorzugsweise um γ-Al₂O₃ oder η-Al₂O₃, und zwar insbesondere dann, wenn neben Aluminiumoxid keine weiteren Metalloxide zugegen sind. Neben Aluminiumoxid können nämlich grundsätzlich auch noch weitere Metalloxide zugegen sein, insbesondere Magnesiumoxid (MgO) in einem auf die Gesamtmasse der Metalloxide bezogenen Massenanteil von 1,0 % bis 60,0 %, bevorzugt 2,0 % bis 50,0 %, besonders bevorzugt 5,0 % bis 35,0 % enthält. Weiterhin kann der erfindungsgemäß einzusetzende Katalysator SiO₂ in einem auf seine Gesamtmasse bezogenen Massenanteil von 1,0 % bis 30,0 %, bevorzugt 2,0 % bis 20,0 %, besonders bevorzugt 2,0 % bis 10,0 % enthalten.

Was die Reaktionsbedingungen betrifft, so kann die Decarboxylierung in einem breiten Bereich von Temperatur und Druck durchgeführt werden. Als Reaktionstemperatur eignet sich vorzugsweise eine Temperatur im Bereich von 150 °C bis 300 °C, besonders bevorzugt 160 °C bis 280 °C, ganz besonders bevorzugt 180 °C bis 240 °C. Der (absolute) Reaktionsdruck kann dabei 0,05 bar bis 300 bar, bevorzugt 1,0 bar bis 100 bar, besonders bevorzugt 1,0 bar bis 60 bar, betragen.

In einer Ausführungsform der Erfindung erfolgt die Decarboxylierung in Gegenwart von Anilin, d. h. die Aminobenzoesäure wird in Anilin gelöst. Bei diskontinuierlicher Durchführung der Reaktion wird vorzugsweise *vor Beginn* der Decarboxylierung ein Massenanteil an Anilin, bezogen auf die Gesamtmasse an Anilin und Aminobenzoesäure, von 0,1 % bis 90 %, bevorzugt 1,0 % bis 70 %, besonders bevorzugt von 5,0 % bis 50 % eingestellt. Bei kontinuierlicher Durchführung der Reaktion wird während der Decarboxylierung *stets* ein Massenanteil an Anilin, bezogen auf die Gesamtmasse an Anilin und Aminobenzoesäure, von 0,1 % bis 90 %, bevorzugt 1,0 % bis 70 %, besonders bevorzugt 5,0 % bis 50 % eingestellt.

Neben Anilin können natürlich auch andere Lösungsmittel bzw. Verdünnungsmittel eingesetzt werden, insbesondere Wasser. Darüber hinaus eignen sich vorzugsweise organische, polare bzw. protische Lösungsmittel wie beispielsweise halogenierte aliphatische oder aromatische Kohlenwasserstoffe, lineare oder cyclische Ether, lineare oder cyclische Ester, lineare oder cyclische Amide, Alkohole, Ketone, Nitrile, Phenolderivate, Benzanilide, Sulfonamide oder Sulfolan, die bevorzugt einen Siedepunkt aufweisen, der bei den gewählten Bedingungen höher ist als die gewählte Reaktionstemperatur und bei dieser mit den Reaktionskomponenten bevorzugt ein homogenes Reaktionsgemisch ausbildet.

Was die Reaktionsführung betrifft, eignet sich sowohl eine Durchführung in der Gasphase als auch in der Flüssigphase. Die Reaktion kann kontinuierlich (bevorzugt) oder diskontinuierlich durchgeführt werden.

Bevorzugte Verfahrensweisen umfassen die Durchführung der Decarboxylierung der Aminobenzoesäure
- in der Flüssig- oder Gasphase in einem Reaktor, insbesondere in einem Rohrreaktor, mit einem integrierten Festbett des Katalysators (beinhaltend eine Schüttung des Katalysators als Formkörper (Extrudate) oder eine Ausgestaltung des Katalysators als monolithische Struktur),
- in der Flüssig- oder - bevorzugt - Gasphase in einem Wirbelschichtreaktor oder
- in der Flüssigphase in einem Rührkessel mit einer darin enthaltenen Suspension *(Slurry)* des Katalysators.

Im Rahmen der vorliegenden Erfindung wird unter einem *Rohrreaktor* ein röhrenförmiger Reaktor verstanden, der bei kontinuierlicher Reaktionsführung (die bevorzugt ist) im Betrieb von dem reagierenden Reaktionsgemisch durchströmt wird. Rohrreaktoren werden bei kleinen Verhältnissen von Länge zu Durchmesser auch als *Turmreaktoren* bezeichnet; auch diese sind vom erfindungsgemäßen Verständnis des Begriffs Rohrreaktor umfasst.

Die Verwendung von Katalysator-Formköpern (Extrudaten) oder monolithischen Katalysator-Strukturen erlaubt eine einfache Wiederverwendung des Katalysators nach erfolgter Decarboxylierung. *"Nach erfolgter Decarboxylierung"* meint im Falle einer diskontinuierlichen Verfahrensführung nach Erreichen des maximalen Umsatzes einer umzusetzenden Charge an Aminobenzoesäure. Im Falle einer kontinuierlichen Verfahrensführung meint *"nach erfolgter Decarboxylierung"* den Zeitpunkt, an dem der Umsatz signifikant unter den anfänglichen Umsatz (der Umsatz zu Beginn eines neuen Reaktionszyklus mit neuem oder regeneriertem Katalysator) sinkt (insbesondere auf einen Wert von 97,0 % oder weniger des anfänglichen Umsatzes sinkt) und die Reaktion daher nicht weiter fortgeführt wird. Zur Umsatzbestimmung eignen sich alle in der Fachwelt bekannten Methoden für die Überwachung kontinuierlich durchgeführter Reaktionen, insbesondere Hochleistungsflüssigkeitschromatographie (HPLC) oder Gaschromatographie (GC). Die gängigen Methoden liefern im Allgemeinen im Rahmen der Messgenauigkeit übereinstimmende Ergebnisse. Sollten wider Erwarten verschiedene Methoden signifikant unterschiedliche Ergebnisse für den Umsatz zu einem bestimmten Zeitpunkt ergeben, so ist für die Zwecke der vorliegenden Erfindung die Umsatzbestimmung über Hochleistungsflüssigkeitschromatographie (HPLC) maßgeblich.

Der nach erfolgter Decarboxylierung vorliegende Katalysator wird vor dem erneuten Einsatz regeneriert. Hierzu kann der Katalysator mit organischem Lösemittel oder wässrigen Lösungen gewaschen werden und / oder bei erhöhter Temperatur in Anwesenheit von O₂ ausgebrannt werden, um organische Ablagerungen zu entfernen.

Das gebildete Anilin kann mit Standardtechniken isoliert und gereinigt werden, insbesondere durch Destillation.

Das auf diese Weise gewonnene Anilin kann in **Schritt (III)** vielfältige Folgeanwendungen unter Ausbildung eines Anilinfolgeprodukts zugeführt werden. Beispielhaft seien die folgenden Umsetzungen genannt:
(1) Säurekatalysierte Reaktion des Anilins mit Formaldehyd unter Bildung von Di- und Polyaminen der Diphenylmethanreihe;
(2) Säurekatalysierte Reaktion des Anilins mit Formaldehyd unter Bildung von Di- und Polyaminen der Diphenylmethanreihe, gefolgt von deren Umsetzung mit Phosgen unter Bildung von Di- und Polyisocyanaten der Diphenylmethanreihe;
   oder
(3) Umsetzung des Anilins zu einer Azoverbindung.

Die weitere Umsetzung Anilins mit Formaldehyd zu **Di- und Polyaminen der Diphenylmethanreihe (III)(1)** ist an sich bekannt und kann nach einem beliebigen Verfahren des Standes der Technik durchgeführt werden. Die kontinuierliche oder teilweise diskontinuierliche Herstellung von Di- und Polyaminen der Diphenylmethanreihe aus Anilin und Formaldehyd ist z. B. in EP 1 616 890 A1, US-A-5286760, EP-A-451442 und WO-A-99/40059 offenbart. Die Reaktion erfolgt unter Säurekatalyse. Als saurer Katalysator eignet sich vorzugsweise Salzsäure.

Die weitere Umsetzung der so erhaltenen Di- und Polyamine der Diphenylmethanreihe mit Phosgen zu **Di- und Polyisocyanaten der Diphenylmethanreihe (III)(2)** ist ebenfalls an sich bekannt und kann nach einem beliebigen Verfahren des Standes der Technik durchgeführt werden. Geeignete Verfahren sind beispielsweise in EP 2 077 150 B1, EP 1 616 857 A1, EP 1 873 142 A1, und EP 0 314 985 B1 beschrieben.

Die Umsetzung des erfindungsgemäß erhaltenen Anilins zu **Azoverbindungen, insbesondere zu Azo-Farbstoffen (III)(3)** kann ebenfalls nach einem beliebigen Verfahren des Standes der Technik erfolgen. Beispielhaft sei auf die bekannte Herstellung von Anilingelb (para-Aminoazobenzol; CAS 493-5-7) oder Indigo (2,2'-Bis(2,3-dihydro-3-oxomethyliden); CAS 482-89-3) verwiesen.

Die Erfindung wird anhand der nachfolgenden Beispiele noch näher erläutert.

### Beispiele:

### Eingesetzte Verbindungen:

### Edukte:

Anthranilsäure (AS, petrochemisch): C₇H₇NO₂, Reinheit ≥ 98 %, Acros Organics
Anthranilsäure (AS, biogenisch): C₇H₇NO₂, Reinheit: 98 %, Covestro Deutschland AG
Anilin (ANL): C₆H₇N, Reinheit ≥ 99.5%, Sigma-Aldrich
2-Aminobenzanilid (AMD): C₁₃H₁₂N₂O, Reinheit 95%, abcr GmbH
VE Wasser: entionisiert
Katalysatoren:
TiO₂ (Anatas, ST61120), SAINT-GOBAIN
SiO₂ (SS61138), SAINT-GOBAIN
Mesostrukturiertes SiO₂ (MCM-41, - 1000 m²/g BET-SA, Sigma-Aldrich)
ZrO₂ (tetragonal, SZ61152), SAINT-GOBAIN
W-dotiertes ZrO₂ (SZ61143), SAINT-GOBAIN
ZnO (≥ 99%), Sigma-Aldrich
Pural Zn44 (44 % Zn in Al₂O₃), Sasol
MgO (Reinheit - 98 %), Acros Organics
Pural MG5 (Mischoxid, Massenverhältnis MgO : Al₂O₃ 5 : 95), Sasol
Pural MG20 (Massenverhältnis MgO : Al₂O₃ 20 : 80), Sasol
Pural MG30 (spinellartige Struktur, Massenverhältnis MgO : Al₂O₃ 30 : 70), Sasol
Al₂O₃ (Al 4126 E), BASF
γ-Al₂O₃ (SA6175), SAINT-GOBAIN
Silylated γ-Al₂O₃ (SA6175), SAINT-GOBAIN
Pural TH100-AlO(OH), Sasol
η-Al₂O₃ (Eta-Alox V1900)
Siralox 5 (Massenverhältnis SiO₂ : Al₂O₃ 5 : 95), Sasol
Siralox10 (Massenverhältnis SiO₂:Al₂O₃ 10 : 90), Sasol
Siralox30 (Massenverhältnis SiO₂ : Al₂O₃ 30 : 70), Sasol
Zeolite CBV600 (Na₂O · Al₂O₃ · SiO₂, Massenverhältnis SiO₂/Al₂O₃ = 5,2; enthält 0,2 Massen-% Na₂O), Zeolyst International.
Puralox SCFa-160/Ce20, 160 m²/g BET-SA, Sasol
Puralox SCFa-190/Zr20, 190 m²/g BET-SA, Sasol
Puralox TH100-150/Ti10, 150 m²/g BET-SA, Sasol
Puralox TH100/150/L4, 150 m²/g BET-SA, Sasol

### Katalysatorvorbereitung:

Alle Katalysatoren für Reaktionen unter Slurry-Bedingungen wurden vor ihrem Einsatz gesiebt (45-90 µm) und für 3 h bei 10 mbar und 200 °C getrocknet. Anschließend wurden die Katalysatoren bis zu ihrem Einsatz unter Ar-Atmosphäre gelagert.

### Methodenbeschreibung:

**HPLC:** Für die HPLC Analyse wurde ein Setup der Firma Agilent mit UV-Detektion (DAD, gemessen bei 254 nm) verwendet. Zur Trennung wurde eine Säule von Agilent (Eclipse XDB-C18; 5 µm; 4.6*150 mm) eingesetzt. Als Fließmittel wurde eine Mischung aus MeOH und Puffer verwendet (Volumenverhältnis MeOH:Puffer = 40:60, Puffer: 0.7 mL 85 %ige p.A. H3PO4 wird auf Endvolumen von 1L mit HPLC Wasser verdünnt, wobei vor der Endauffüllung der pH Wert von 3.0 mit Natronlauge einzustellen ist). Die Flußrate betrug 0.7 mL/min. Der Säulenofen wurde auf 40 °C temperiert. Das Injektionsvolumen betrug 1 µL. Die Retentionszeiten der Einzelkomponenten Anilin (ANL), Anthranilsäure (AA) und 2-Aminobenzanilid (ABD) waren: ANL = 3.2 min; AA = 5.2 min; Amid = 15.7 min.

Die Peakflächen werden in Flächenprozent (A%) umgerechnet. Die Quantifizierung der Einzelkomponenten in Massenprozent (wt.-%), bezogen auf das Reaktionsgemisch, wurde durch eine zuvor erstellte Kalibrierung mit Reinsubstanzen ermöglicht. Neben der Massenzusammensetzung wird auf Basis der Werte der Umsatz an Anthranilsäure, die Ausbeute an gebildetem Anilin, die Selektivität der Anilinbildung und die Selektivität zur Bildung von 2-Aminobenzanilid bestimmt.

### Allgemeine Arbeitsvorschrift 1: Decarboxylierung von Anthranilsäure (Slurry; Bsp. 1 bis 62)

Die Decarboxylierung von Anthranilsäure wird in einem Stahlreaktor durchgeführt, der mit 1,6 g Anthranilsäure, Anilin (optional, Menge siehe Tabelle 1), pulverförmigem Katalysator (optional, Menge siehe Tabelle 2) und VE Wasser (optional, Menge siehe Tabelle 1) befüllt wird. Anschließend wird der Stahlreaktor verschlossen und mit Ar gespült. Das Reaktionsgemisch wird anschließend für eine definierte Reaktionszeit bei definierter Temperatur und 360 U/min gerührt. Hierbei baut sich zunehmend Druck durch die Freisetzung von CO₂ auf. Anschließend wir das Reaktionsgemisch im Eisbad abgekühlt, der Druck abgelassen und das Gemisch und mit 4,0 g Methanol verdünnt. Das verdünnte Gemisch wird filtriert und mittels HPLC-Analytik charakterisiert.

### Allgemeine Arbeitsvorschrift 2: Decarboxylierung von Anthranilsäure (Extrudate; Bsp. 63 bis 74)

Zur Decarboxylierung von Anthranilsäure mit Extrudaten werden Al₂O₃-Extrudate (BASF; Al 4126 E) in zylindrischer Form (Querschnitt-Durchmesser ≈ 3 mm, Länge ≈ 4,5 mm) verwendet und die Recyclingfähigkeit über 6 Katalysator-Recycling-Zyklen untersucht. Dazu werden 1,6 g Anthranilsäure in einen Autoklaven mit einem Edelstahlkäfig gegeben, der 0,2 g Al₂O₃-Extrudate enthält. Anschließend werden 0,25 g destilliertes Wasser (13,5 Gew.-%) zugegeben. Nach Absenken des Drucks auf 100 mbar und Spülen des Reaktors mit Ar-Gas wird das Reaktionsgemisch auf 225 °C aufgeheizt und für 1 h gerührt (360 U/min). Hierbei baut sich zunehmend Druck durch die Freisetzung von CO₂ auf. Anschließend wir das Reaktionsgemisch im Eisbad abgekühlt, der Druck abgelassen und das Gemisch mit ca. 4,0 g Methanol verdünnt. Das verdünnte Gemisch wird mittels HPLC-Analytik charakterisiert. Anschließend werden die verwendeten Extrudate für den nächsten Reaktionszyklus eingesetzt.

Die Versuche werden in den nachfolgenden Tabellen zusammengefasst. Darin werden die folgenden Abkürzungen verwendet:
- V =: Vergleichsbeispiel
- Kat. =: Katalysator
- ANL =: Anilin
- AS =: Anthranilsäure
- AMD =: 2-Aminobenzanilid

**Tabelle 1: Metalloxid-Zusammensetzungen der getesteten Katalysatoren**

| Katalysator | | Al₂O₃ | MgO | SiO₂ | TiO₂ | ZnO | ZrO₂ | CeO₂ | La₂O₃ |
|---|---|---|---|---|---|---|---|---|---|
| Nr. | Bezeichnung | Massenanteil in %, bezogen auf die Gesamtmasse aller im Material vorhanden Metalloxide | | | | | | | |
| K1 | TiO₂ (Anatas) | 0 | 0 | 0 | 100 | 0 | 0 | 0 | 0 |
| K2 | SiO₂ | 0 | 0 | 100 | 0 | 0 | 0 | 0 | 0 |
| K3 | MCM41/SiO₂ | 0 | 0 | 100 | 0 | 0 | 0 | 0 | 0 |
| K4 | ZrO₂ | 0 | 0 | 0 | 0 | 0 | 100 | 0 | 0 |
| K5 | W-dotiertes ZrO₂ | 0 | 0 | 0 | 0 | 0 | ≥90 | 0 | 0 |
| K6 | ZnO | 0 | 0 | 0 | 0 | 100 | 0 | 0 | 0 |
| K7 | MgO | 0 | 100 | 0 | 0 | 0 | 0 | 0 | 0 |
| K8 | Zeolith CBV 600 | 24,6 | 0 | 75,4 | 0 | 0 | 0 | 0 | 0 |
| K9 | η-Al₂O₃ | 100 | 0 | 0 | 0 | 0 | 0 | 0 | 0 |
| K10 | γ-Al₂O₃ | 100 | 0 | 0 | 0 | 0 | 0 | 0 | 0 |
| K11 | γ-Al₂O₃;silyliert | 100 | 0 | 0 | 0 | 0 | 0 | 0 | 0 |
| K12 | Al₂O₃ (BASF, Al 4126 E) | 100 | 0 | 0 | 0 | 0 | 0 | 0 | 0 |
| K13 | TH100-AlO(OH) (Böhmit) | 100 | 0 | 0 | 0 | 0 | 0 | 0 | 0 |
| K14 | Pural^{®} ZN44 | 56 | 0 | 0 | 0 | 44 | 0 | 0 | 0 |
| K15 | Pural^{®} MG5 | 95 | 5 | 0 | 0 | 0 | 0 | 0 | 0 |
| K16 | Pural^{®} MG20 | 80 | 20 | 0 | 0 | 0 | 0 | 0 | 0 |
| K17 | Pural^{®} MG30 | 70 | 30 | 0 | 0 | 0 | 0 | 0 | 0 |
| K19 | Siralox^{®} 5 | 95 | 0 | 5 | 0 | 0 | 0 | 0 | 0 |
| K20 | Siralox^{®} 10 | 90 | 0 | 10 | 0 | 0 | 0 | 0 | 0 |
| K21 | Siralox^{®} 30 | 70 | 0 | 30 | 0 | 0 | 0 | 0 | 0 |
| K22 | Puralox^{®} SCFa-160/ Ce20 | 79,7 | 0 | 0 | 0 | 0 | 0 | 20,3 | 0 |
| K23 | Puralox^{®} SCFa-190/Zr20 | 79,6 | 0 | 0 | 0 | 0 | 20,4 | 0 | 0 |
| K24 | Puralox ^{®}TH100-150/Ti10 | 89,6 | 0 | 0 | 10,4 | 0 | 0 | 0 | 0 |
| K25 | Puralox^{®} TH100/150/L4 | 96,0 | 0 | 0 | 0 | 0 | 0 | 0 | 4,0 |

**Tabelle 2: Decarboxylierung von Anthranilsäure zu Anilin. Reaktionstemperatur T = 185 °C; Reaktionsdauer t_{R} = 20 min. Wechselnde Katalysatoren.**

| Bsp. | **Kat.[a]** | | ASₜ₌ₜ₀ [b] | ANL_{t**=**t0} [b] | H₂O [b] | ANL_{t**=**t**R**} [c] | AS_{t=tR} [c] | AMD_{t=tR} [c] | A_{ANL} [d] | X_{AS} [e] | S_{ANL} [f] | S_{AMD} [f] |
|---|---|---|---|---|---|---|---|---|---|---|---|---|
| 1 (V) | - | 0 | 50,0 | 50,0 | 0 | 57,5 | 41,7 | 0,8 | 17,1 | 19,0 | 90,0 | 5,0 |
| 2 (V) | **K1** | 5,9 | 50,0 | 50,0 | 0 | 58,1 | 41,0 | 0,9 | 18,2 | 20,4 | 89,4 | 5,3 |
| 3 (V) | **K2** | 5,9 | 50,0 | 50,0 | 0 | 59,4 | 39,6 | 1,0 | 21,3 | 23,7 | 89,7 | 5,1 |
| 4 (V) | **K3** | 5,9 | 50,0 | 50,0 | 0 | 61,4 | 37,5 | 1,1 | 25,3 | 28,0 | 90,4 | 4,8 |
| 5 (V) | **K4** | 5,9 | 50,0 | 50,0 | 0 | 59,8 | 39,3 | 0,8 | 21,8 | 23,9 | 91,4 | 4,3 |
| 6 (V) | **K5** | 5,9 | 50,0 | 50,0 | 0 | 65,1 | 34,1 | 0,8 | 32,9 | 34,9 | 94,1 | 3,0 |
| 7 (V) | **K6** | 5,9 | 50,0 | 50,0 | 0 | 67,2 | 32,3 | 0,4 | 38,1 | 39,2 | 97,2 | 1,4 |
| 8 (V) | **K7** | 5,9 | 50,0 | 50,0 | 0 | 70,3 | 29,2 | 0,6 | 44,4 | 45,8 | 97,1 | 1,5 |
| 9(V) | **K8** | 5,9 | 50,0 | 50,0 | 0 | 71,2 | 28,1 | 0,7 | 46,2 | 47,8 | 96,6 | 1,7 |
| 10 | **K9** | 5,9 | 50,0 | 50,0 | 0 | 94,7 | 5,0 | 0,3 | 90,8 | 91,5 | 99,2 | 0,4 |
| 11 | **K10** | 5,9 | 50,0 | 50,0 | 0 | 94,3 | 5,2 | 0,5 | 90,0 | 91,1 | 98,8 | 0,6 |
| 12 | **K11** | 5,9 | 50,0 | 50,0 | 0 | 95,5 | 4,1 | 0,4 | 92,1 | 93,0 | 99,1 | 0,5 |
| 13 | **K12** | 5,9 | 50,0 | 50,0 | 0 | 88,4 | 11,1 | 0,5 | 79,5 | 80,5 | 98,6 | 0,7 |
| 13a | **K12** | 5,9 | 50,0 [g] | 50,0 | 0 | 92,5 | 7,2 | 0,3 | 86,8 | 87,5 | 99,2 | 0,4 |
| 14 | **K13** | 5,9 | 50,0 | 50,0 | 0 | 89,0 | 10,4 | 0,6 | 80,5 | 81,9 | 98,3 | 0,9 |
| 15 | **K14** | 5,9 | 50,0 | 50,0 | 0 | 73,7 | 25,8 | 0,6 | 51,2 | 52,6 | 97,4 | 1,3 |
| 16 | **K15** | 5,9 | 50,0 | 50,0 | 0 | 95,6 | 4,2 | 0,1 | 92,5 | 92,8 | 99,7 | 0,2 |
| 17 | **K16** | 5,9 | 50,0 | 50,0 | 0 | 96,8 | 3,1 | 0,1 | 94,6 | 95,0 | 99,8 | 0,1 |
| 18 | **K17** | 5,9 | 50,0 | 50,0 | 0 | 96,5 | 3,4 | 0,1 | 94,0 | 94,2 | 99,7 | 0,1 |
| 18a | **K17** | 5,9 | 50,0 [g] | 50,0 | 0 | 97,0 | 2,9 | 0,1 | 94,9 | 95,1 | 99,8 | 0,1 |
| 20 | **K19** | 5,9 | 50,0 | 50,0 | 0 | 95,7 | 4,0 | 0,3 | 92,5 | 93,1 | 99,4 | 0,3 |
| 21 | **K20** | 5,9 | 50,0 | 50,0 | 0 | 95,4 | 4,3 | 0,3 | 92,0 | 92,7 | 99,3 | 0,4 |
| 22 | **K21** | 5,9 | 50,0 | 50,0 | 0 | 77,8 | 21,4 | 0,9 | 59,3 | 61,3 | 96,8 | 1,6 |
| 23 | **K22** | 5,9 | 50,0 | 50,0 | 0 | 82,6 | 16,9 | 0,6 | 68,7 | 70,0 | 98,1 | 1,0 |
| 24 | **K23** | 5,9 | 50,0 | 50,0 | 0 | 74,0 | 25,3 | 0,7 | 51,6 | 53,3 | 96,8 | 1,6 |
| 25 | **K24** | 5,9 | 50,0 | 50,0 | 0 | 76,2 | 23,2 | 0,6 | 56,4 | 57,9 | 97,5 | 1,3 |
| 26 | **K25** | 5,9 | 50,0 | 50,0 | 0 | 74,5 | 24,9 | 0,6 | 52,9 | 54,3 | 97,4 | 1,3 |

**Tabelle 3: Decarboxylierung von Anthranilsäure zu Anilin. Reaktionstemperatur T = 185 °C. Wechselnde Eduktmischungszusammensetzungen.**

| Bsp. | Kat. [a] | | **ASₜ₌ₜ₀ [b]** | **ANLₜ₌ₜ₀ [b]** | H₂O [b] | t_{R} / min | ANL_{t**=** tR} [c] | AS_{t=tR} [c] | AMD_{t=tR} [c] | A_{ANL} [d] | X_{AS} [e] | S_{ANL} [f] | S_{AMD} [f] |
|---|---|---|---|---|---|---|---|---|---|---|---|---|---|
| 27 | - | 0 | **50,0** | **50,0** | 0 | 60 | 75,2 | 22,8 | 1,9 | 53,9 | 58,5 | 92,2 | 3,9 |
| 28 | K17 | 5,9 | **50,0** | **50,0** | 0 | 60 | 98,4 | 1,5 | 0,1 | 97,2 | 97,4 | 99,7 | 0,1 |
| 29 (V) | - | 0 | **60,0** | **40,0** | 0 | 60 | 75,0 | 23,0 | 2,2 | 61,8 | 66,0 | 94,0 | 3,1 |
| 30 | K17 | 6,9 | **60,0** | **40,0** | 0 | 60 | 98,2 | 1,7 | 0,1 | 97,5 | 97,7 | 99,7 | 0,1 |
| 31 (V) | - | 0 | **72,0** | **28,0** | 0 | 60 | 72,8 | 24,7 | 2,5 | 67,7 | 71,4 | 94,8 | 2,6 |
| 32 | K17 | 8,3 | **72,0** | **28,0** | 0 | 60 | 97,1 | 2,7 | 0,2 | 96,9 | 97,1 | 99,7 | 0,1 |
| 33 (V) | - | 0 | **88,0** | **12,0** | 0 | 60 | 70,4 | 26,9 | 2,7 | 72,9 | 76,0 | 95,9 | 2,1 |
| 34 | K17 | 10 | **88,0** | **12,0** | 0 | 60 | 91,0 | 8,6 | 0,4 | 92,5 | 92,9 | 99,5 | 0,2 |
| 35 (V) | - | 0 | **100** | **0** | 0 | 60 | 70,0 | 27,1 | 3,0 | 77,0 | 80,0 | 96,4 | 1,8 |
| 36 | K17 | 11 | **100** | **0** | 0 | 60 | 85,2 | 14,0 | 0,8 | 89,3 | 90,0 | 99,2 | 0,4 |
| 37 | K12 | 20 | **100** | **0** | 0 | 180 | 98,1 | 1,0 | 0,9 | 98,5 | 99,3 | 99,2 | 0,4 |
| 38 | K12 | 5,9 | **100** | **0** | 0 | 180 | 97,2 | 0,6 | 2,2 | 97,6 | 99,6 | 98,0 | 1,0 |

**Tabelle 4: Decarboxylierung von Anthranilsäure zu Anilin. Reaktionstemperatur T = 185 °C Reaktionsdauer t_{R} = 20 min. Wechselnde Wassergehalte.**

| Bsp. | Kat. [a] | | ASₜ₌ₜ₀ [b] | ANL_{t=t**0**} [b] | **H₂O [b]** | ANL_{t**=**t**R**} [c] | AS_{t=tR} [c] | AMD_{t=tR} [c] | A_{ANL} [d] | X_{AS} [e] | S_{ANL} [f] | S_{AMD} [f] |
|---|---|---|---|---|---|---|---|---|---|---|---|---|
| 39 (V) | - | 0 | 48,0 | 48,0 | **5,0** | 58,6 | 40,7 | 0,7 | 18,9 | 20,7 | 91,2 | 4,4 |
| 40 (V) | - | 0 | 45,0 | 45,0 | **10,0** | 61,1 | 38,3 | 0,6 | 25,1 | 26,5 | 94,6 | 2,7 |
| 41 (V) | - | 0 | 43,0 | 43,0 | **13,0** | 63,4 | 36,2 | 0,4 | 30,1 | 31,1 | 96,6 | 1,7 |
| 42 | K12 | 5,6 | 48,0 | 48,0 | **5,0** | 94,5 | 5,3 | 0,2 | 90,5 | 90,9 | 99,5 | 0,2 |
| 43 | K12 | 5,4 | 45,0 | 45,0 | **10,0** | 94,0 | 5,9 | 0,1 | 89,5 | 89,8 | 99,6 | 0,2 |
| 44 | K12 | 5,2 | 43,0 | 43,0 | **13,0** | 94,8 | 5,1 | 0,1 | 90,9 | 91,2 | 99,7 | 0,1 |
| 45 | K10 | 5,6 | 48,0 | 48,0 | **5,0** | 94,5 | 5,4 | 0,1 | 90,4 | 90,7 | 99,7 | 0,2 |
| 46 | K10 | 5,4 | 45,0 | 45,0 | **10,0** | 95,3 | 4,7 | 0,1 | 91,8 | 92,0 | 99,8 | 0,1 |
| 47 | K10 | 5,2 | 43,0 | 43,0 | **13,0** | 95,6 | 4,3 | 0,1 | 92,5 | 92,7 | 99,8 | 0,1 |
| 48 | K17 | 5,6 | 48,0 | 48,0 | **5,0** | 96,7 | 3,2 | 0,05 | 94,4 | 94,6 | 99,9 | 0,06 |
| 49 | K17 | 5,4 | 45,0 | 45,0 | **10,0** | 96,0 | 4,0 | 0,04 | 93,1 | 93,2 | 99,9 | 0,05 |
| 50 | K17 | 5,2 | 43 | 43 | **13** | 93,6 | 6,4 | 0,02 | 89,0 | 89,1 | 99,9 | 0,03 |

**Tabelle 5: Decarboxylierung von Anthranilsäure zu Anilin. Wechselnde Wassergehalte, Reaktionstemperaturen und -dauern, sowie unterschiedliche Anthranilsäure-Quellen mit K17 bzw. K12.**

| Bsp. | Kat. [a] | | ASₜ₌ₜ₀ [b] | AN L_{t=t 0} [b] | **H₂O [b]** | **T / °C** | **t_{R} / min** | ANL_{t=t R} [c] | A_{St=t R} [c] | AMD_{t=t R} [c] | A_{ANL} [d] | X_{AS} [e] | S_{ANL} [f] | S_{AMD} [f] |
|---|---|---|---|---|---|---|---|---|---|---|---|---|---|---|
| 51 | K17 | 5,9 | 50 | 50 | **0** | **185** | **60** | 98,9 | 1,0 | 0,1 | 98,1 | 98,3 | 99,8 | 0,1 |
| 52 | K17 | 5,6 | 48 | 48 | **5,0** | **185** | **60** | 98,9 | 1,1 | 0,04 | 98,1 | 98,2 | 99,9 | 0,05 |
| 53 | K17 | 5,9 | 50 | 50 | **0** | **200** | **20** | 98,7 | 1,2 | 0,1 | 97,8 | 97,9 | 99,8 | 0,1 |
| 54 | K17 | 5,9 | 50 | 50 | **0** | **200** | **60** | 99,1 | 0,8 | 0,1 | 98,4 | 98,7 | 99,8 | 0,1 |
| 55 | K17 | 5,6 | 48 | 48 | **5,0** | **200** | **60** | 99,3 | 0,6 | 0,1 | 98,8 | 98,9 | 99,9 | 0,06 |
| 56 | K17 | 5,9 | 50 | 50 | **0** | **225** | **20** | 98,7 | 1,2 | 0,1 | 97,8 | 98,0 | 99,8 | 0,1 |
| 57 | K17 | 5,9 | 50 | 50 | **0** | **225** | **60** | 99,2 | 0,7 | 0,1 | 98,6 | 98,9 | 99,7 | 0,1 |
| 57a | K17 | 5,9 | 50 [g] | 50 | **0** | **225** | **60** | 97,9 | 1,9 | 0,2 | 96,4 | 96,8 | 99,6 | 0,2 |
| 58 | K17 | 5,6 | 48 | 48 | **5,0** | **225** | **60** | 99,2 | 0,7 | 0,1 | 98,6 | 98,8 | 99,8 | 0,1 |
| 59 | K17 | 5,9 | 50 | 50 | **0** | **230** | **20** | 98,8 | 1,1 | 0,1 | 98,0 | 98,2 | 99,8 | 0,1 |
| 60 | K17 | 5,9 | 50 | 50 | **0** | **230** | **60** | 99,2 | 0,7 | 0,1 | 98,6 | 98,9 | 99,7 | 0,1 |
| 61 | K17 | 5,6 | 48 | 48 | **5,0** | **230** | **60** | 99,0 | 0,9 | 0,1 | 98,4 | 98,5 | 99,8 | 0,1 |
| 62 | K12 | 9,7 | 86,5 | 0 | 13,5 | 225 | 60 | 99,5 | 0,2 | 0,3 | 99,6 | 99,9 | 99,7 | 0,1 |
| 62a | K12 | 9,7 | 86,5 [g] | 0 | 13,5 | 225 | 60 | 98,9 | 0,6 | 0,5 | 99,2 | 99,6 | 99,5 | 0,2 |

**Tabelle 6: Decarboxylierung von Anthranilsäure zu Anilin ohne Verdünnung mit ANL. Einsatz des Katalysators als Extrudat und Wiederverwertung desselben.**

| Bsp. | Kat. [h] | | ASₜ₌ₜ₀ [b] | ANLₜ₌ₜ₀ [b] | **H₂O [b]** | **T / °C** | **t_{R} / min** | ANL_{t=tR} [c] | AS_{t=tR} [c] | AMD_{t=tR} [c] | A_{ANL} [d] | X_{AS} [e] | S_{ANL} [f] | S_{AMD} [f] |
|---|---|---|---|---|---|---|---|---|---|---|---|---|---|---|
| 63 | K12 | 9,7 | 86,5 | 0 | 13,5 | 225 | 60 | 98,3 | 0,5 | 1,2 | 98,6 | 99,6 | 98,9 | 0,5 |
| 64 | K12 | 9,7 | 86,5 | 0 | 13,5 | 225 | 60 | 98,2 | 0,7 | 1,1 | 98,6 | 99,5 | 99,0 | 0,5 |
| 65 | K12 | 9,7 | 86,5 | 0 | 13,5 | 225 | 60 | 98,8 | 0,1 | 1,1 | 98,9 | 99,9 | 99,1 | 0,5 |
| 66 | K12 | 9,7 | 86,5 | 0 | 13,5 | 225 | 60 | 98,7 | 0,4 | 0,9 | 98,9 | 99,7 | 99,2 | 0,4 |
| 67 | K12 | 9,7 | 86,5 | 0 | 13,5 | 225 | 60 | 98,7 | 0,4 | 0,9 | 98,9 | 99,7 | 99,2 | 0,4 |
| 68 | K12 | 9,7 | 86,5 | 0 | 13,5 | 225 | 60 | 99,0 | 0,2 | 0,7 | 99,2 | 99,8 | 99,4 | 0,3 |
| 69 | K12 | 9,7 | 86,5 | 0 | 13,5 | 225 | 60 | 99,0 | 0,1 | 0,9 | 99,1 | 99,9 | 99,2 | 0,4 |
| 70 | K12 | 9,7 | 86,5 | 0 | 13,5 | 225 | 60 | 98,8 | 0,3 | 0,9 | 99,0 | 99,8 | 99,2 | 0,4 |
| 71 | K12 | 9,7 | 86,5 | 0 | 13,5 | 225 | 60 | 98,7 | 0,5 | 0,9 | 98,9 | 99,7 | 99,2 | 0,4 |
| 72 | K12 | 9,7 | 86,5 | 0 | 13,5 | 225 | 60 | 98,9 | 0,3 | 0,8 | 99,1 | 99,8 | 99,3 | 0,3 |
| 73 | K12 | 9,7 | 86,5 | 0 | 13,5 | 225 | 60 | 98,6 | 0,4 | 1,0 | 98,9 | 99,7 | 99,1 | 0,4 |
| 74 | K12 | 9,7 | 86,5 | 0 | 13,5 | 225 | 60 | 99,1 | 0,3 | 0,6 | 99,2 | 99,8 | 99,5 | 0,3 |

### Erläuterungen zu den Tabellen:

[a] Massenanteil in % basierend auf der Summe von AS, ANL, H₂O u. Kat.; Katalysator wird als Pulver eingesetzt (Slurry);
[b] Massenanteil in % in der Eduktmischung, bezogen auf Gesamtmasse von AS, ANL und H₂O;
[c] Massenanteil in % in der Produktmischung, bezogen auf Gesamtmasse von AS, ANL und 2-Aminobenzanilid;
[d] Chemische Ausbeute an Anilin in %;
[e] Chemischer Umsatz an Anthranilsäure in %
[f] Selektivität zu Anilin bzw. 2-Aminobenzanilid in %;
[g] Fermentativ hergestellte Anthranilsäure;
[h] Massenanteil in % basierend auf der Summe von AS, ANL, H₂O u. Kat.; Katalysator wird als Extrudat eingesetzt. In den Beispielen 63 bis 74 wurde jeweils der Katalysator des vorangegangenen Beispiels wiederverwendet. Beispiel 74 stellt daher den zwölften Verwendungszyklus im Sinne eines Katalysator-Recyclings über mehrere Versuche dar.

Wie die Beispiele zeigen, ermöglicht das erfindungsgemäße Verfahren die Umsetzung von Aminobenzoesäure mit hohen Umsätzen bei geringer Nebenproduktbildung was zu einer hohen Ausbeute an Anilin führt. Die erfindungsgemäßen Katalysatoren ermöglichen die Bildung von Anilin in sehr hohen Ausbeuten bei variabler Zusammensetzung des ANL/AA-Substratgemisches, des Wassergehalts und einem variablen Prozessfenster in Bezug auf Reaktionszeit und -temperatur. Es wurde der technische Effekt in Bezug auf erhöhte Anilinausbeute mit den erfindungsgemäßen Katalysatoren gegenüber dem Stand der Technik gezeigt. Der Katalysator kann als Slurry oder extrudierter Festkörper eingesetzt werden. Langzeitstabilität des Katalysators konnte ohne merklichen Abfall der Anilinausbeute über 12 Zyklen nachgewiesen werden. Die erfindungsgemäßen Katalysatoren eignen sich für die Decarboxylierung von petrochemischer hergestellter Anthranilsäure sowie biogen basierter Anthranilsäure.

## Patentansprüche

1. Verfahren zur Herstellung von Anilin oder eines Anilinfolgeproduktes, umfassend die Schritte:
(I) Bereitstellen von Aminobenzoesäure;
(II) Decarboxylieren der Aminobenzoesäure zu Anilin in Gegenwart eines anorganischen heterogenen Metalloxid-Katalysators enthaltend einen auf die Gesamtmasse der Metalloxide bezogenen Massenanteil an Al₂O₃ von 40,0 % bis 100 %, wobei der auf die Gesamtmasse des anorganischen heterogenen Metalloxid-Katalysators bezogene Massenanteil an Al₂O₃ 25 % bis 100 % beträgt;
und
(III) optional, Umsetzen des Anilins zu einem Anilinfolgeprodukt.

2. Verfahren nach Anspruch 1, bei welchem der anorganische heterogene Metalloxid-Katalysator MgO in einem auf die Gesamtmasse der Metalloxide bezogenen Massenanteil von 1,0 % bis 60,0 % enthält.

3. Verfahren nach Anspruch 1 oder 2, bei welchem der anorganische heterogene Metalloxid-Katalysator SiO₂ in einem auf seine Gesamtmasse bezogenen Massenanteil von 1,0 % bis 30,0 % enthält.

4. Verfahren nach einem der vorstehenden Ansprüche, bei welchem das Al₂O₃ γ-Al₂O₃ oder η-Al₂O₃ umfasst.

5. Verfahren nach einem der vorstehenden Ansprüche, bei welchem das Decarboxylieren der Aminobenzoesäure bei einer Temperatur von 150 °C bis 300 °C durchgeführt wird.

6. Verfahren nach Anspruch 5, bei welchem das Decarboxylieren der Aminobenzoesäure bei einem absoluten Druck von 0,05 bar bis 300 bar durchgeführt wird.

7. Verfahren nach einem der vorstehenden Ansprüche, bei welchem das Decarboxylieren der Aminobenzoesäure in Gegenwart von Anilin durchgeführt wird.

8. Verfahren nach Anspruch 7, bei welchem das Decarboxylieren der Aminobenzoesäure diskontinuierlich durchgeführt und vor Beginn der Decarboxylierung ein Massenanteil an Anilin, bezogen auf die Gesamtmasse an Anilin und Aminobenzoesäure, von 0,1 % bis 90 % eingestellt wird.

9. Verfahren nach Anspruch 7, bei welchem das Decarboxylieren der Aminobenzoesäure kontinuierlich durchgeführt und während der Decarboxylierung stets ein Massenanteil an Anilin, bezogen auf die Gesamtmasse an Anilin und Aminobenzoesäure, von 0,1 % bis 90 % eingestellt wird.

10. Verfahren nach einem der vorstehenden Ansprüche, bei welchem das Decarboxylieren der Aminobenzoesäure
• in der Flüssig- oder Gasphase in einem Reaktor mit einem integrierten Festbett des anorganischen heterogenen Metalloxid-Katalysators,
• in der Flüssig- oder Gasphase in einem Wirbelschichtreaktor oder
• in der Flüssigphase in einem Rührkessel mit einer darin enthaltenen Suspension des anorganischen heterogenen Metalloxid-Katalysators
durchgeführt wird.

11. Verfahren nach Anspruch 10, bei welchem das Decarboxylieren der Aminobenzoesäure in der Flüssig- oder Gasphase in einem Reaktor mit einem integrierten Festbett des anorganischen heterogenen Metalloxid-Katalysators beinhaltend eine Schüttung des Katalysators als Formkörper oder eine Ausgestaltung des Katalysators als monolithische Struktur durchgeführt wird, wobei der anorganische heterogene Metalloxid-Katalysator nach erfolgter Decarboxylierung regeneriert und wiederverwendet wird.

12. Verfahren nach einem der vorstehenden Ansprüche, bei welchem Schritt (I) das Fermentieren eines Rohstoffes enthaltend eine fermentierbare Kohlenstoff-haltige Verbindung und eine Stickstoff-haltige Verbindung in Gegenwart von Mikroorganismen umfasst.

13. Verfahren nach Anspruch 12, bei welchem die Mikroorganismen *Escherichia coli, Pseudomonas putida, Corynebacterium glutamicum, Ashbya gossypii, Pichia pastoris, Hansenula polymorpha, Yarrowia lipolytica, Zygosaccharomyces bailii* oder *Saccharomyces cerevisiae* umfassen.

14. Verfahren nach einem der vorstehenden Ansprüche, bei welchem Schritt (III) durchgeführt wird und eine der folgenden Umsetzungen umfasst:
(1) Säurekatalysierte Reaktion des Anilins mit Formaldehyd unter Bildung von Di- und Polyaminen der Diphenylmethanreihe;
(2) Säurekatalysierte Reaktion des Anilins mit Formaldehyd unter Bildung von Di- und Polyaminen der Diphenylmethanreihe, gefolgt von deren Umsetzung mit Phosgen unter Bildung von Di- und Polyisocyanaten der Diphenylmethanreihe;
oder
(3) Umsetzung des Anilins zu einer Azoverbindung.

15. Verfahren gemäß einem der vorstehenden Ansprüche, bei welchem die in Schritt (A) bereitgestellte Aminobenzoesäure ortho-Aminobenzoesäure umfasst.

## Claims

1. Process for preparing aniline or an aniline conversion product, comprising the steps of:
(I) providing aminobenzoic acid;
(II) decarboxylating the aminobenzoic acid to aniline in the presence of an inorganic heterogeneous metal oxide catalyst containing a proportion by mass of Al₂O₃ based on the total mass of the metal oxides of 40.0% to 100%, the proportion by mass of Al₂O₃ based on the total mass of the inorganic heterogeneous metal oxide catalyst being 25% to 100%;
and
(III) optionally converting the aniline to an aniline conversion product.

2. Process according to Claim 1, in which the inorganic heterogeneous metal oxide catalyst contains MgO in a proportion by mass based on the total mass of the metal oxides of 1.0% to 60.0%.

3. Process according to Claim 1 or 2, in which the inorganic heterogeneous metal oxide catalyst contains SiO₂ in a proportion by mass based on its total mass of 1.0% to 30.0%.

4. Process according to any of the preceding claims, in which the Al₂O₃ comprises γ-Al₂O₃ or η-Al₂O₃.

5. Process according to any of the preceding claims, in which the decarboxylation of the aminobenzoic acid is performed at a temperature of 150°C to 300°C.

6. Process according to Claim 5, in which the decarboxylation of the aminobenzoic acid is performed at an absolute pressure of 0.05 bar to 300 bar.

7. Process according to any of the preceding claims, in which the decarboxylation of the aminobenzoic acid is performed in the presence of aniline.

8. Process according to Claim 7, in which the decarboxylation of the aminobenzoic acid is performed batchwise and a proportion by mass of aniline, based on the total mass of aniline and aminobenzoic acid, of 0.1% to 90% is established before the start of the decarboxylation.

9. Process according to Claim 7, in which the decarboxylation of the aminobenzoic acid is performed continuously and a proportion by mass of aniline, based on the total mass of aniline and aminobenzoic acid, of 0.1% to 90% is always established during the decarboxylation.

10. Process according to any of the preceding claims, in which the decarboxylation of the aminobenzoic acid is performed
• in the liquid or gas phase in a reactor with an integrated fixed bed of the inorganic heterogeneous metal oxide catalyst,
• in the liquid or gas phase in a fluidized bed reactor or
• in the liquid phase in a stirred tank with a suspension of the inorganic heterogeneous metal oxide catalyst contained therein.

11. Process according to Claim 10, in which the decarboxylation of the aminobenzoic acid is performed in the liquid or gas phase in a reactor with an integrated fixed bed of the inorganic heterogeneous metal oxide catalyst containing a bed of the catalyst as shaped bodies or a configuration of the catalyst as a monolithic structure, the inorganic heterogeneous metal oxide catalyst being regenerated and reused on completion of decarboxylation.

12. Process according to any of the preceding claims, in which step (I) comprises the fermentation of a raw material containing a fermentable carbon-containing compound and a nitrogen-containing compound in the presence of microorganisms.

13. Process according to Claim 12, in which the microorganisms comprise *Escherichia coli, Pseudomonas putida, Corynebacterium glutamicum, Ashbya gossypii, Pichia pastoris, Hansenula polymorpha, Yarrowia lipolytica, Zygosaccharomyces bailii* or *Saccharomyces cerevisiae.*

14. Process according to any of the preceding claims, in which step (III) is performed and comprises one of the following conversions:
(1) acid-catalyzed reaction of the aniline with formaldehyde to form di- and polyamines of the diphenylmethane series;
(2) acid-catalyzed reaction of the aniline with formaldehyde to form di- and polyamines of the diphenylmethane series, followed by reaction thereof with phosgene to form di- and polyisocyanates of the diphenylmethane series;
or
(3) conversion of the aniline to an azo compound.

15. Process according to any of the preceding claims, in which the aminobenzoic acid provided in step (A) comprises ortho-aminobenzoic acid.

## Revendications

1. Procédé de préparation d'aniline ou d'un dérivé d'aniline, comprenant les étapes :
(I) mise à disposition d'acide aminobenzoïque ;
(II) décarboxylation de l'acide aminobenzoïque en aniline en présence d'un catalyseur hétérogène inorganique à oxydes métalliques contenant une proportion massique d'Al₂O₃ de 40,0 % à 100 % par rapport à la masse totale des oxydes métalliques, la proportion massique d'Al₂O₃ par rapport à la masse totale du catalyseur hétérogène inorganique à oxydes métalliques étant de 25 % à 100 % ;
et
(III) éventuellement, transformation de l'aniline en un dérivé d'aniline.

2. Procédé selon la revendication 1, dans lequel le catalyseur hétérogène inorganique à oxydes métalliques contient du MgO en une proportion massique de 1,0 % à 60,0 % par rapport à la masse totale des oxydes métalliques.

3. Procédé selon la revendication 1 ou 2, dans lequel le catalyseur hétérogène inorganique à oxydes métalliques contient du SiO₂ en une proportion massique de 1,0 % à 30,0 % par rapport à sa masse totale.

4. Procédé selon l'une des revendications précédentes, dans lequel Al₂O₃ comprend du γ-Al₂O₃ ou du η-Al₂O₃.

5. Procédé selon l'une des revendications précédentes, dans lequel la décarboxylation de l'acide aminobenzoïque est réalisée à une température de 150 °C à 300 °C.

6. Procédé selon la revendication 5, dans lequel la décarboxylation de l'acide aminobenzoïque est réalisée à une pression absolue de 0,05 bar à 300 bars.

7. Procédé selon l'une des revendications précédentes, dans lequel la décarboxylation de l'acide aminobenzoïque est réalisée en présence d'aniline.

8. Procédé selon la revendication 7, dans lequel la décarboxylation de l'acide aminobenzoïque est réalisée de manière discontinue et, avant le début de la décarboxylation, la proportion massique d'aniline est réglée à 0,1 % jusqu'à 90 %, par rapport à la masse totale d'aniline et d'acide aminobenzoïque.

9. Procédé selon la revendication 7, dans lequel la décarboxylation de l'acide aminobenzoïque est réalisée de manière continue et, pendant la décarboxylation, la proportion massique d'aniline est toujours réglée à 0,1 % jusqu'à 90 %, par rapport à la masse totale d'aniline et d'acide aminobenzoïque.

10. Procédé selon l'une des revendications précédentes, dans lequel la décarboxylation de l'acide aminobenzoïque est réalisée
• en phase liquide ou gazeuse dans un réacteur à lit fixe intégré du catalyseur hétérogène inorganique à oxydes métalliques,
• en phase liquide ou gazeuse dans un réacteur à lit tourbillonnant ou
• en phase liquide dans une cuve agitée contenant en son sein une suspension du catalyseur hétérogène inorganique à oxydes métalliques.

11. Procédé selon la revendication 10, dans lequel la décarboxylation de l'acide aminobenzoïque est réalisée en phase liquide ou gazeuse dans un réacteur à lit fixe intégré du catalyseur hétérogène inorganique à oxydes métalliques comprenant une masse en vrac du catalyseur sous forme de corps moulés ou une conception du catalyseur en tant que structure monolithique, le catalyseur hétérogène inorganique à oxydes métalliques étant régénéré et réutilisé après la décarboxylation.

12. Procédé selon l'une des revendications précédentes, dans lequel l'étape (i) comprend la fermentation d'une matière première contenant un composé fermentable contenant du carbone et un composé contenant de l'azote en présence de micro-organismes.

13. Procédé selon la revendication 12, dans lequel les micro-organismes comprennent *Escherichia coli, Pseudomonas putida, Corynebacterium glutamicum, Ashbya gossypii, Pichia pastoris, Hansenula polymorpha, Yarrowia lipolytica, Zygosaccharomyces bailii* ou *Saccharomyces cerevisiae.*

14. Procédé selon l'une des revendications précédentes, dans lequel l'étape (III) est réalisée et comprend l'une transformations suivantes :
(1) réaction catalysée par un acide de l'aniline avec du formaldéhyde avec formation de diamines et de polyamines de la série du diphénylméthane ;
(2) réaction catalysée par un acide de l'aniline avec du formaldéhyde avec formation de diamines et de polyamines de la série du diphénylméthane, suivie de leur transformation avec du phosgène avec formation de diisocyanates et de polyisocyanates de la série du diphénylméthane ;
ou
(3) réaction de l'aniline en un composé azoïque.

15. Procédé selon l'une des revendications précédentes, dans lequel l'acide aminobenzoïque mis à disposition dans l'étape (A) comprend de l'acide ortho-aminobenzoïque.
